# EUROPEAN PATENT APPLICATION

(11) **EP 3 103 472 A1**
(43) Date of publication of application: **14.12.2016**
(21) Application number: 16180406.7
(22) Date of filing: 22.03.2013
(51) Int. Cl.: A61K 39/08, A61K 8/64, A61P 17/00, A61F 13/00, A61Q 19/08, A61K 8/02, A61K 8/99

(54) **METHOD OF TREATMENT OF WRINKLES USING TOPICAL CHEMODENERVATING AGENTS**

(30) Priority: 22.03.2012 US 201261614206 P
(62) Divisional of application: 13764725.1
(71) Applicant: ReVance Therapeutics, Inc., Newark, CA 94560 (US)
(72) Inventor: WAUGH, Jacob, San Francisco, CA California 94119 (US); BROWNE, L., Daniel, Palo Alto, CA California 94301 (US)
(74) Representative: Wright, Simon Mark

(57) **Abstract**

Methods for reducing the appearance of wrinkles in a subject are provided herein. The methods of the present invention comprise identifying a wrinkle distribution on a subject and applying a topical composition comprising at least one chemodenervating agent onto and along the wrinkle distribution. The methods disclosed herein provide alternative methods for delivery of chemodenervating agents to the skin for the treatment of wrinkles.

SEQUENCE LISTING

<110> WAUGH, JACOB M.
      BROWNE, L. DANIEL

<120> METHOD OF TREATMENT OF WRINKLES USING TOPICAL CHEMODENERVATING
      AGENTS

<130> 13720-105144US1

<140> 13/848,786
<141> 2013-03-22

<150> 61/614,206
<151> 2012-03-22

<160> 18

<170> PatentIn version 3.5

<210> 1
<211> 45
<212> PRT
<213> Artificial Sequence

<220>
<223> Description of Artificial Sequence: Synthetic
      polypeptide


<220>
<221> misc_feature
<222> (1)..(20)
<223> This region may encompass 0-20 residues

<220>
<221> misc_feature
<222> (21)..(45)
<223> This region may encompass 5, 7, 9, 11, 13, 15, 17, 19, 21
      23 or 25 residues

<220>
<223> See specification as filed for detailed description of
      substitutions and preferred embodiments

<400> 1
Gly Gly Gly Gly Gly Gly Gly Gly Gly Gly Gly Gly Gly Gly Gly Gly
1               5                   10                  15


Gly Gly Gly Gly Arg Arg Arg Arg Arg Arg Arg Arg Arg Arg Arg Arg
            20                  25                  30


Arg Arg Arg Arg Arg Arg Arg Arg Arg Arg Arg Arg Arg
        35                  40                  45


<210> 2
<211> 51
<212> PRT
<213> Artificial Sequence

<220>
<223> Description of Artificial Sequence: Synthetic
      polypeptide

<220>
<221> misc_feature
<222> (1)..(20)
<223> This region may encompass 0-20 residues

<220>
<221> misc_feature
<222> (32)..(51)
<223> This region may encompass 0-20 residues

<220>
<223> See specification as filed for detailed description of
      substitutions and preferred embodiments

<400> 2
Gly Gly Gly Gly Gly Gly Gly Gly Gly Gly Gly Gly Gly Gly Gly Gly
1               5                   10                  15

Gly Gly Gly Gly Arg Gly Arg Asp Asp Arg Arg Gln Arg Arg Arg Gly
            20                  25                  30

Gly Gly Gly Gly Gly Gly Gly Gly Gly Gly Gly Gly Gly Gly Gly Gly
        35                  40                  45

Gly Gly Gly
    50

<210> 3
<211> 51
<212> PRT
<213> Artificial Sequence

<220>
<223> Description of Artificial Sequence: Synthetic
      polypeptide

<220>
<221> misc_feature
<222> (1)..(20)
<223> This region may encompass 0-20 residues

<220>
<221> misc_feature
<222> (32)..(51)
<223> This region may encompass 0-20 residues

<220>
<223> See specification as filed for detailed description of
      substitutions and preferred embodiments

<400> 3
Gly Gly Gly Gly Gly Gly Gly Gly Gly Gly Gly Gly Gly Gly Gly Gly
1               5                   10                  15

Gly Gly Gly Gly Tyr Gly Arg Lys Lys Arg Arg Gln Arg Arg Arg Gly
            20                  25                  30

Gly Gly Gly Gly Gly Gly Gly Gly Gly Gly Gly Gly Gly Gly Gly Gly
        35                  40                  45

Gly Gly Gly
    50

<210> 4
<211> 49
<212> PRT
<213> Artificial Sequence

<220>
<223> Description of Artificial Sequence: Synthetic
      polypeptide

<220>
<221> misc_feature
<222> (1)..(20)
<223> This region may encompass 0-20 residues

<220>
<221> misc_feature
<222> (30)..(49)
<223> This region may encompass 0-20 residues

<220>
<223> See specification as filed for detailed description of
      substitutions and preferred embodiments

<400> 4
Gly Gly Gly Gly Gly Gly Gly Gly Gly Gly Gly Gly Gly Gly Gly Gly
1               5                   10                  15

Gly Gly Gly Gly Arg Lys Lys Arg Arg Gln Arg Arg Arg Gly Gly Gly
            20                  25                  30

Gly Gly Gly Gly Gly Gly Gly Gly Gly Gly Gly Gly Gly Gly Gly Gly
        35                  40                  45

Gly

<210> 5
<211> 35
<212> PRT
<213> Artificial Sequence

<220>
<223> Description of Artificial Sequence: Synthetic
      polypeptide

<400> 5
Arg Lys Lys Arg Arg Gln Arg Arg Arg Gly Lys Lys Lys Lys Lys Lys
1               5                   10                  15

Lys Lys Lys Lys Lys Lys Lys Lys Lys Gly Arg Lys Lys Arg Arg Gln
            20                  25                  30

Arg Arg Arg
        35

<210> 6
<211> 40
<212> PRT
<213> Artificial Sequence

<220>
<223> Description of Artificial Sequence: Synthetic
      polypeptide

<400> 6
Arg Lys Lys Arg Arg Gln Arg Arg Arg Gly Lys Lys Lys Lys Lys Lys
1               5                   10                  15

Lys Lys Lys Lys Lys Lys Lys Lys Lys Lys Lys Lys Lys Lys Gly Arg
            20                  25                  30

Lys Lys Arg Arg Gln Arg Arg Arg
        35                  40

<210> 7
<211> 45
<212> PRT
<213> Artificial Sequence

<220>
<223> Description of Artificial Sequence: Synthetic
      polypeptide

<400> 7
Arg Lys Lys Arg Arg Gln Arg Arg Arg Gly Lys Lys Lys Lys Lys Lys
1               5                   10                  15

Lys Lys Lys Lys Lys Lys Lys Lys Lys Lys Lys Lys Lys Lys Lys Lys
            20                  25                  30

Lys Lys Lys Gly Arg Lys Lys Arg Arg Gln Arg Arg Arg
        35                  40                  45

<210> 8
<211> 50
<212> PRT
<213> Artificial Sequence

<220>
<223> Description of Artificial Sequence: Synthetic
      polypeptide

<400> 8
Arg Lys Lys Arg Arg Gln Arg Arg Arg Gly Lys Lys Lys Lys Lys Lys
1               5                   10                  15

Lys Lys Lys Lys Lys Lys Lys Lys Lys Lys Lys Lys Lys Lys Lys Lys
            20                  25                  30

Lys Lys Lys Lys Lys Lys Lys Lys Gly Arg Lys Lys Arg Arg Gln Arg
        35                  40                  45

Arg Arg
    50


<210> 9
<211> 39
<212> PRT
<213> Artificial Sequence

<220>
<223> Description of Artificial Sequence: Synthetic
      polypeptide

<400> 9
Arg Gly Arg Asp Asp Arg Arg Gln Arg Arg Arg Gly Lys Lys Lys Lys
1               5                   10                  15

Lys Lys Lys Lys Lys Lys Lys Lys Lys Lys Lys Gly Arg Gly Arg Asp
            20                  25                  30

Asp Arg Arg Gln Arg Arg Arg
        35


<210> 10
<211> 44
<212> PRT
<213> Artificial Sequence

<220>
<223> Description of Artificial Sequence: Synthetic
      polypeptide

<400> 10
Arg Gly Arg Asp Asp Arg Arg Gln Arg Arg Arg Gly Lys Lys Lys Lys
1               5                   10                  15

Lys Lys Lys Lys Lys Lys Lys Lys Lys Lys Lys Lys Lys Lys Lys Lys
            20                  25                  30

Gly Arg Gly Arg Asp Asp Arg Arg Gln Arg Arg Arg
        35                  40

<210> 11
<211> 49
<212> PRT
<213> Artificial Sequence

<220>
<223> Description of Artificial Sequence: Synthetic
      polypeptide

<400> 11
Arg Gly Arg Asp Asp Arg Arg Gln Arg Arg Arg Gly Lys Lys Lys Lys
1               5                   10                  15

Lys Lys Lys Lys Lys Lys Lys Lys Lys Lys Lys Lys Lys Lys Lys Lys
            20                  25                  30

Lys Lys Lys Lys Lys Gly Arg Gly Arg Asp Asp Arg Arg Gln Arg Arg
        35                  40                  45

Arg

<210> 12
<211> 54
<212> PRT
<213> Artificial Sequence

<220>
<223> Description of Artificial Sequence: Synthetic
      polypeptide

<400> 12
Arg Gly Arg Asp Asp Arg Arg Gln Arg Arg Arg Gly Lys Lys Lys Lys
1               5                   10                  15

Lys Lys Lys Lys Lys Lys Lys Lys Lys Lys Lys Lys Lys Lys Lys Lys
            20                  25                  30

Lys Lys Lys Lys Lys Lys Lys Lys Lys Lys Gly Arg Gly Arg Asp Asp
        35                  40                  45

Arg Arg Gln Arg Arg Arg
    50

<210> 13
<211> 39
<212> PRT
<213> Artificial Sequence

<220>
<223> Description of Artificial Sequence: Synthetic
      polypeptide

<400> 13
Tyr Gly Arg Lys Lys Arg Arg Gln Arg Arg Arg Gly Lys Lys Lys Lys
1               5                   10                  15

Lys Lys Lys Lys Lys Lys Lys Lys Lys Lys Lys Gly Tyr Gly Arg Lys
            20                  25                  30

Lys Arg Arg Gln Arg Arg Arg
        35

<210> 14
<211> 44
<212> PRT
<213> Artificial Sequence

<220>
<223> Description of Artificial Sequence: Synthetic
      polypeptide

<400> 14
Tyr Gly Arg Lys Lys Arg Arg Gln Arg Arg Arg Gly Lys Lys Lys Lys
1               5                   10                  15

Lys Lys Lys Lys Lys Lys Lys Lys Lys Lys Lys Lys Lys Lys Lys Lys
            20                  25                  30

Gly Tyr Gly Arg Lys Lys Arg Arg Gln Arg Arg Arg
        35                  40

<210> 15
<211> 49
<212> PRT
<213> Artificial Sequence

<220>
<223> Description of Artificial Sequence: Synthetic
      polypeptide

<400> 15
Tyr Gly Arg Lys Lys Arg Arg Gln Arg Arg Arg Gly Lys Lys Lys Lys
1               5                   10                  15

Lys Lys Lys Lys Lys Lys Lys Lys Lys Lys Lys Lys Lys Lys Lys Lys
            20                  25                  30

Lys Lys Lys Lys Lys Gly Tyr Gly Arg Lys Lys Arg Arg Gln Arg Arg
        35                  40                  45

Arg

<210> 16
<211> 54

<212> PRT
<213> Artificial Sequence

<220>
<223> Description of Artificial Sequence: Synthetic
      polypeptide

<400> 16
Tyr Gly Arg Lys Lys Arg Arg Gln Arg Arg Arg Gly Lys Lys Lys Lys
1               5                   10                  15

Lys Lys Lys Lys Lys Lys Lys Lys Lys Lys Lys Lys Lys Lys Lys Lys
            20                  25                  30

Lys Lys Lys Lys Lys Lys Lys Lys Lys Lys Gly Tyr Gly Arg Lys Lys
        35                  40                  45

Arg Arg Gln Arg Arg Arg
    50

<210> 17
<211> 10
<212> PRT
<213> Artificial Sequence

<220>
<223> Description of Artificial Sequence: Synthetic
      peptide

<400> 17
Gly Gly Gly Arg Arg Arg Arg Arg Arg Arg
1               5                   10

<210> 18
<211> 25
<212> PRT
<213> Artificial Sequence

<220>
<223> Description of Artificial Sequence: Synthetic
      peptide

<220>
<221> misc_feature
<222> (1)..(8)
<223> This region may encompass 1-8 residues

<220>
<221> misc_feature
<222> (9)..(25)
<223> This region may encompass 7, 9, 11, 13, 15 or 17
      residues

<400> 18
Gly Gly Gly Gly Gly Gly Gly Gly Arg Arg Arg Arg Arg Arg Arg Arg
1               5                   10                  15

Arg Arg Arg Arg Arg Arg Arg Arg Arg
            20                  25

## Description

### FIELD OF THE INVENTION

This invention relates to methods of treating wrinkles using topical compositions comprising chemodenervating agents, including methods of applying topical chemodenervating agents to treat wrinkles.

### BACKGROUND OF THE INVENTION

Botulinum toxin is an extremely potent neurotoxin produced by the bacteria *Clostridium botulinum*. The toxin acts as a chemodenervating agent by inhibiting the release of the neurotransmitter acetylcholine, thereby preventing synaptic transmission across the neuromuscular junction and inhibiting muscular contraction to cause temporary paralysis.

Historically, botulinum toxin has been used for the correction of neurological and neuromuscular disorders, such as hemifacial spasm, adult onset spasmodic torticollis, anal fissure, blepharospasm, cerebral palsy, cervical dystonia, migraine headaches, and strabismus. More recently, botulinum toxin has proven useful for certain dermatologic and cosmetic indications, such as the management of hyperhidrosis, facial rhytides (wrinkles), and other disorders resulting from spasms or from contractions of facial muscles.

While the use of botulinum toxin to treat wrinkles is currently one of the most popular cosmetic treatments, the conventional method of administering toxin for this purpose by injecting the toxin into a patient gives rise to several problems. First, botulinum toxin typically must be injected into multiple sites in order to treat a given wrinkle. The selection of the particular sites of injection is not easy and must be determined by a skilled practitioner with a deep understanding of muscle anatomy. The injections, which are performed along the muscle or muscles responsible for forming the wrinkle (rather than along the wrinkle itself), must be done with proper technique. Improper injection technique can lead to undesirable effects, including the unintended spread of the toxin away from the injection site and to adjacent muscles, thereby weakening the muscle and affecting facial expression or function. Eyelid ptsosis (drooping eyelid), for example, can result when improper injection technique is used when treating forehead lines.

Furthermore, the multiple injections required to treat a single wrinkle can be painful, and injections can result in bruising and/or irritation around the injection site. The pain or anticipated pain associated with the injections can lead to anxiousness, stress or embarrassment in patients, thereby affecting their quality of life. Moreover, an entire patient population that could potentially benefit from the use of such chemodenervating agents remains untreated due to severe needle-related phobias or aversions.

Moreover, the effects of most chemodenervating agents, such as botulinum toxin, are temporary. The effects of injected botulinum toxin typically last between three and six months, after which the paralyzed nerve recovers and re-innervates the muscle by forming new nerve branches. Therefore, as the paralysis subsides, a patient is faced with the prospect of undergoing additional painful injections. With the current technology, a patient must receive periodic injections indefinitely in order to achieve and maintain the desired results.

Accordingly, there is a need for improved methods of administering potent chemodenervating agents, such as botulinum toxin, for treating wrinkles. Specifically, there is a need for an efficacious, less painful method of delivering chemodenervating agents such as botulinum toxin to a patient for reducing the appearance of wrinkles.

### SUMMARY OF THE INVENTION

This invention relates to methods of treating wrinkles using topical compositions comprising chemodenervating agents. The method of treating wrinkles includes identifying a wrinkle distribution on a subject's skin. The wrinkle distribution may comprise one or more wrinkles. A composition comprising one or more chemodenervating agents is topically applied onto and along the wrinkle distribution to reduce the appearance of one or more wrinkles in the wrinkle distribution. Optionally, an occlusive dressing may be applied to the treated area of the subject's skin.

### BRIEF DESCRIPTION OF THE FIGURES

**FIG. 1****:** Schematic diagram of a prior art injection pattern for injecting botulinum toxin in order to treat glabellar lines.
**FIG. 2****:** Schematic diagram of a prior art injection pattern for injecting botulinum toxin in order to treat glabellar lines.
**FIG. 3****:** Schematic diagram of a prior art injection pattern for injecting botulinum toxin in order to treat glabellar lines.

### DETAILED DESCRIPTION OF THE INVENTION

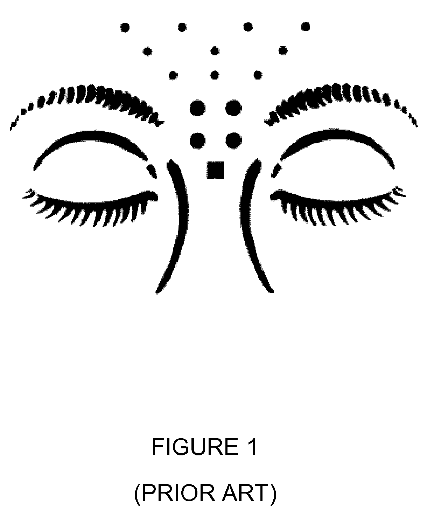

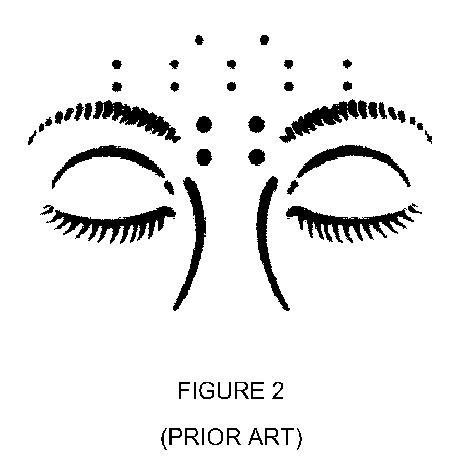

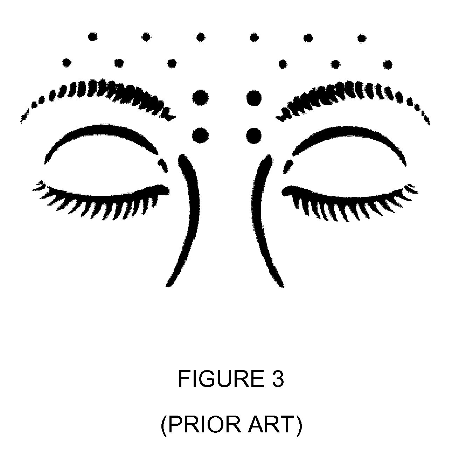

Injecting botulinum toxin to treat facial wrinkles involves identifying the facial muscle or muscles responsible for causing the wrinkles and selecting multiple injection sites along the muscle or muscles. Once the multiple injection sites have been selected, a clinician must carefully inject the botulinum toxin specifically at those sites, taking care to administer the proper amount of botulinum toxin at each site. FIGS. 1-3 show exemplary injection site profiles for treating lateral canthal lines, as discussed in a previous study (*see* Letessier et al., "Treatment of wrinkles with botulinum toxin," J. Derm. Treat. (1999) 10, 31-36, the contents of which are incorporated by reference in their entirety). As shown in FIG. 1, this injection pattern requires botulinum toxin to be injected into fifteen sites distributed over three different muscles: (1) the procerus muscle (where the injection site is represented by a single square); (2) the corrugator muscle (where the injection sites are represented by four large circles); and (3) the frontalis muscle (where the injection sites are represented by ten small circles). FIG. 2 shows a different injection pattern involving injection of botulinum toxin into sixteen sites distributed over two different muscles: (1) the corrugator muscle (where the injection sites are represented by four large circles); and (2) the frontalis muscle (where the injection sites are represented by twelve small circles). A third injection pattern is provided by FIG. 3, which requires sixteen injections distributed over two different muscles: (1) the corrugator muscle (where the injection sites are represented by four large circles); and (2) the frontalis muscle (where the injection sites are represented by twelve small circles).

Note that in each of the foregoing injection patterns in the prior art, the injection sites for treating lateral canthal lines are selected based on the underlying muscle anatomy, rather than on the position of the lateral canthal lines themselves. For instance, the injection sites for the frontalis muscle as depicted in FIGS. 1-3 spread across the forehead, instead of being isolated in the glabellar region, where lateral canthal lines are found. Indeed, in such injection patterns, injection sites may correspond to areas where there is no skin wrinkling associated with lateral canthal lines.

The present invention is based, at least in part, on the surprising discovery that topically applied chemodenervating agents for treating wrinkles need not be applied based on the anatomy of the underlying muscles that are responsible for causing wrinkles in the skin. In other words, the invention recognizes that the protocol previously used for injecting botulinum toxin to treat wrinkles (*viz*., identifying muscles responsible for the wrinkles, selecting multiple administration sites based on the muscle anatomy, and administering the botulinum toxin according to the selected administration sites) is not necessary when topical administration is used.

In one aspect, the invention provides methods for topically administering chemodenervating agents, such as botulinum toxin, in order to reduce the appearance of wrinkles. In certain embodiments, the method for reducing the appearance of wrinkles comprises identifying a wrinkle distribution on a subject's skin and then applying a topical composition comprising a chemodenervating agent directly to and along the wrinkle distribution to reduce the appearance of the one or more wrinkles in the wrinkle distribution.

As used herein, the term "wrinkle" refers to a fold or crease in the skin. Wrinkles can vary in size and intensity, from fine lines to deep furrows. Fine wrinkles encompass "crinkles" as well as lines which have a shallow trough and typically do not have significant ridging; ridging refers to the raising of the wrinkle edge above the adjacent plane of unwrinkled skin; fine wrinkles typically do not cast a shadow from light illuminated at an angle across the wrinkle. Deep wrinkles tend to have both a trough which is below the plane of the adjacent skin as well as ridging which extends above the plane of the adjacent unwrinkled skin. Deep wrinkles typically can cast shadows when illuminated with an appropriate light source at an angle across the wrinkle. Wrinkles in skin may be classified into three different types: dynamic wrinkles, static wrinkles and wrinkle folds. Dynamic wrinkles are caused by repeated contractions of muscles underlying the skin. For example, frowning or furrowing causes wrinkles between the eyebrows (glabellar lines or "frown lines"), raising of the eyebrows causes the horizontal lines alone the forehead (forehead rows) and smiling and/or squinting causes wrinkles at the corners of the eyes (lateral canthal lines or "crow's feet"). Static wrinkles result from a loss of elasticity in skin, which may arise from a variety of factors, including sun damage, poor nutrition, smoking, and genetic factors. Wrinkle folds, which may appear as deep grooves between the nose and mouth, arise from the sagging of underlying facial structure. Generally, the methods of this invention are suitable for treatment of all types of wrinkles. In certain embodiments of this invention, the wrinkles that are treated with topical chemodenervating agents are dynamic wrinkles.

As used herein, the term "wrinkle distribution" refers to one or more wrinkles present in a given area on the skin of a subject in need of treatment. Generally speaking, the invention is suitable for treating wrinkle distributions present on any area of skin of a subject in need of treatment. Non-limiting examples of areas that may be treated include the face, head, neck, hands, feet, shoulders, chest, torso and back. In addition, when the area to be treated is the face, the wrinkle distribution may be located in specific subregions of the face, such as the forehead, temples, cheeks, or jawline.

The specific shape of a wrinkle distribution and the number of wrinkles in a wrinkle distribution are not particularly limited. This invention specifically contemplates embodiments in which the wrinkle distribution comprises one wrinkle, two wrinkles, three wrinkles, four wrinkles, or even five or more wrinkles. When the wrinkle distribution comprises two or more wrinkles, some or all of the wrinkles may be contiguous. This invention, however, expressly contemplates wrinkle distributions where at least one wrinkle in the wrinkle distribution is not contiguous with other wrinkles. For example, a wrinkle distribution may include wrinkles that are symmetrically located on either side of the face. Non-limiting types of wrinkles present in the wrinkle distribution that can be reduced in appearance by the methods of the present invention are lateral canthal lines (crow's feet), glabellar lines (vertical lines between the eyebrows), forehead lines, platysma lines (neck lines), nasolabial lines ("smile lines" or "laugh lines) or perioral lip lines (around the mouth and lips).

In certain embodiments, once a wrinkle distribution is identified, a topical composition comprising at least one chemodenervating agent is applied onto and along the wrinkle distribution. In this context, "applied onto and along the wrinkle distribution" refers to directly applying the topical composition along the contours of the one or more wrinkles in the wrinkle distribution. This may be achieved, for example, by using a custom applicator, such as the one described in U.S. Pre-Grant Publication No. 2011/010621 to Ruegg et al., which is hereby incorporated by reference in its entirety. Alternatively, the topical composition may be applied onto and along the wrinkle distribution by first applying the topical composition to an implement such as a swab or even just a gloved finger, and then tracing the contours of the wrinkles in the wrinkle distribution with the swab or gloved finger.

In certain embodiments consistent with the principle of the invention, a topical composition is applied onto and along a wrinkle distribution comprising at least one lateral canthal line, glabellar line, forehead line, platysma line, one nasolabial line, or perioral line. Of course, a wrinkle distribution may contain combinations of such wrinkles. Moreover, in certain embodiments, the topical composition can be applied to multiple wrinkle distributions.

On the other hand, the invention recognizes that a wrinkle distribution in a certain area of skin may be treated by covering the entire area region of skin with a layer of a topical composition comprising at least one chemodenervating agent. This approach is less favored than applying the topical composition onto and along the wrinkle distribution, particularly in cases where the chemodenervating agent is a highly toxic substance (*e.g*., botulinum toxin). For example, this approach may lead to chronic administration of higher doses of chemodenervating agent, which in turn may cause the patient to develop an immunity to the chemodenervating agent.

### Occlusive Dressings

An occlusive dressing optionally may be applied to the area of treatment following application of a topical composition comprising at least one chemodenervating agent. The occlusive dressing may serve one or more purposes, including preventing accidental contact with the area of skin treated with the topical composition, preventing the topically applied composition from the being inadvertent spread to other regions of skin, or helping to remove excess topical composition on the surface of the skin by absorption or adsorption. In certain embodiments, the occlusive dressing prevents the topical composition from prematurely drying out, which may cause a decrease in the transdermal flux of the chemodenervating agent. Thus, in some embodiments, the occlusive dressing remains on the skin for a period of time sufficient to allow an effective amount of the chemodenervating agent to penetrate the skin. As the skilled artisan will appreciate, the period of time that the occlusive dressing remains on a treated area of skin depends several factors, including without limitation the specific formulation of the topical composition, the intended dosage, and the amount that is applied. In this regard, the occlusive dressing may be applied to the treated area of the skin for at least 1, 2, 3, 5, 10, 15, 20, 25, or 30 minutes and at most 45, 50, 60, 65, 70, 75, 80, 90, 100, 120, 150, 180, or 240 minutes, with each specific combination of lower limit and upper limit being an embodiment expressly contemplated by the invention. In one particularly useful embodiment, the occlusive dressing remains on the treated area of skin for about 30 minutes.

The material used as an occlusive dressing is not particularly limited, so long as it serves the purposes set forth herein. Typically, the occlusive dressing is flexible, so that it may conform to the shape of the treated area of skin. In certain preferred embodiments, the portion of the occlusive dressing that contacts the treated area is chemically inert with respect to the topical formulation. The occlusive dressing can be a sheet-like material (e.g., a fabric or a foil), which optionally may be patterned on one side with an adhesive to affix the occlusive dressing to the skin of the patient. In certain embodiments, the occlusive dressing comprises a polymeric material, non-limiting examples of which include cellulosic fibers, polyalkylene chloride, polylactic acid, polyethylene-vinylacetate, Teflon^{®}, polyurethane, polyethylene, polypropylene, polystyrene and combinations or copolymers thereof. The occlusive dressing optionally may include Tegaderm^{™} and Glad Cling Wrap^{™}.

### Chemodenervating Agents

The topical compositions for use with the method for treating wrinkles disclosed herein typically contain at least one chemodenervating agent. The term "chemodenervating agent," as used herein, refers to a substance that prevents a nerve from stimulating its target tissue, *e.g.* a muscle, a gland or another nerve. Generally speaking, chemodenervating agents act by interrupting nerve impulse transmission across a neuromuscular or neuroglandular junction, thereby blocking or reducing neuronal exocytosis of a neurotransmitter, or altering the action potential at a sodium channel voltage gate of a neuron. Non-limiting examples of chemodenervating agents contemplated by the invention include botulinum toxin, tetanus toxin, saxitoxin, and tetrodotoxin, and all serotypes and all combinations thereof.

The term "chemodenervation" encompasses all effects which directly or indirectly are induced by the chemodenervating agent, therefore also comprising upstream, downstream or long-term effects of said chemodenervating agent. Therefore presynaptic effects are also encompassed as well as postsynaptic effects, tissue effects and/or indirect effects via spinal or afferent neurons.

The chemodenervating agent is present in the topical composition in an amount effective to reduce the appearance of the wrinkle distribution in a subject. The reduction in the appearance of wrinkles can be readily determined by one of skill in the art. In one embodiment, wrinkle reduction is determined by comparing the relative smoothness of the surface of the wrinkle distribution before and after treatment with the topical composition, with increased smoothness indicating a reduction in wrinkles. In another embodiment, wrinkle reduction is determined by comparing the appearance of the wrinkle distribution before and after treatment with the topical composition (e.g., by comparing photographs), as is known in the art.

In certain embodiments, the chemodenervating agent is a botulinum toxin. The term "botulinum toxin," as used herein, refers to any of the known types of botulinum toxin, whether produced by the bacterium or by recombinant techniques, as well as any such types that may be subsequently discovered including engineered variants or fusion proteins. The botulinum toxin is selected from the group consisting of serotypes A, B, C, D, E, F, G and combinations thereof. Botulinum toxin is commercially available under a variety of different trade names, including BOTOX™ (Allergan, Inc) and DYSPORT^{™} (Ipsen, Limited) and MYOBLOC^{™} (Elan Pharmaceuticals, Inc.)

In certain embodiments, botulinum toxin is present as an isolated botulinum toxin molecule (*e.g*., botulinum toxin type A neurotoxin) that has been stabilized by exogenous stabilizers. See, *e.g.,* U.S. Pre-Grant Publication 20100330123 entitled "Albumin Free Botulinum Toxin Formulations," which is hereby incorporated by reference in its entirety. Alternatively, the botulinum toxin may be present in a complexed form, stabilized, at least in part, by one or more of the non-toxin, non-hemaglutinin proteins and non-toxin, hemaglutinin proteins that are normally expressed along with the botulinum toxin by the *C*. *Botulinum* bacteria. In certain embodiments, the botulinum toxin is stabilized by exogenous stabilizers, such as albumin.

The chemodenervating agent can alternatively be a derivatized form of a naturally occurring chemodenervating agent. For example, the chemodenervating agent may be a derivatized form of botulinum toxin, that is, a compound that has toxin activity but contains one or more chemical or functional alterations on any part or on any chain relative to naturally occurring or recombinant native toxins. More particularly, the botulinum toxin may be a modified neurotoxin (*e.g*., a neurotoxin which has at least one of its amino acids deleted, modified or replaced, as compared to a native, or a recombinantly produced neurotoxin or a derivative or fragment thereof). Alternatively, the botulinum toxin may be one that has been modified in a way that, for instance, enhances its properties or decreases undesirable side effects, but that still retains the desired botulinum toxin activity. The botulinum toxin may be any of the botulinum toxin complexes produced by the bacterium, as described above. Alternatively, the botulinum toxin may be a toxin prepared using recombinant or synthetic chemical techniques (e.g. a recombinant peptide, a fusion protein, or a hybrid neurotoxin, as prepared from subunits or domains of different botulinum toxin serotypes (see U.S. Pat. No. 6,444,209, for instance)). The botulinum toxin may also be a portion of the overall molecule that has been shown to possess the necessary botulinum toxin activity, and in such case may be used per se or as part of a combination or conjugate molecule, for instance a fusion protein. Additionally, the botulinum toxin may be in the form of a botulinum toxin precursor, which may itself be non-toxic, for instance a nontoxic zinc protease that becomes toxic on proteolytic cleavage.

### Transdermal Carriers

As the skilled artisan will appreciate, certain chemodenervating agents do not pass through skin unassisted. For instance, botulinum toxin type A, as expressed by *C. Botulinum*, typically exists in a 900 kD complex containing the 150 kD neurotoxin along with various endogenous non-toxin accessory. Neither the 900 kD complex, nor the 150 kD neurotoxin alone penetrates intact skin unassisted.

Accordingly, this invention recognizes that the topical compositions for treating wrinkles according to the method described herein may include, in certain embodiments, a transdermal carrier to promote the transport of the chemodenervating agent. Generally, the chemodenervating agent and the transdermal carrier are administered together, preferably without covalent modification of the chemodenervating agent. The transdermal carrier is not particularly limited, and may be any transdermal carrier that permits the application of at least one chemodenervating agent onto and along a wrinkle distribution and that increases the transdermal flux of the chemodenervating agent. For instance, the transdermal carrier may be a liquid chemical carrier, such as dimethyl sulfoxide (DMSO). The transdermal carrier may also be in the form of a nanoemulsion as described in WO2008/045107. In other embodiments, the transdermal carrier may comprise sphingosine and/or cerebroside as described in U.S. Pre-Grant Publication No. 20060182766. The transdermal carrier may also comprise a sialoprotein, such as those described, for example in U.S. Pre-Grant Publication No. 20070116724. It is to be understood that the foregoing references, like all references cited herein, are incorporated by reference in their entirety.

In certain embodiments, the topical compositions according to the invention comprise a transdermal carrier includes a positively charged carrier molecule with positively charged efficiency groups attached thereto, as is disclosed, for example, in U.S. Pre-Grant Publication No. 20080107690. By "positively charged" is meant that the carrier has a positive charge under at least some solution-phase conditions, more preferably under at least some physiologically compatible conditions. More specifically, "positively charged" as used herein, means that the group in question contains functionalities that are charged under all pH conditions, for instance, a quaternary amine, or contains a functionality which can acquire positive charge under certain solution-phase conditions, such as pH changes in the case of primary amines. More preferably, "positively charged" as used herein refers to those groups that have the behavior of associating with anions over physiologically compatible conditions. Polymers with a multiplicity of positively-charged moieties need not be homopolymers, as will be apparent to one skilled in the art. Other examples of positively charged moieties are well known in the prior art and can be employed readily, as will be apparent to those skilled in the art.

In certain embodiments, the positively-charged carrier comprises a "positively charged backbone," which is typically a linear chain of atoms, either with groups in the chain carrying a positive charge at physiological pH, or with groups carrying a positive charge attached to side chains extending from the backbone. Preferably, the positively charged backbone itself will not have a defined enzymatic or therapeutic biologic activity. The linear backbone is a hydrocarbon backbone which is, in some embodiments, interrupted by heteroatoms selected from nitrogen, oxygen, sulfur, silicon and phosphorus. The majority of backbone chain atoms are usually carbon. Additionally, the backbone will often be a polymer of repeating units (e.g., amino acids, poly(ethyleneoxy), poly(propyleneamine), polyalkyleneimine, and the like) but can be a heteropolymer. In one group of embodiments, the positively charged backbone is a polypropyleneamine wherein a number of the amine nitrogen atoms are present as ammonium groups (tetra-substituted) carrying a positive charge. In another embodiment, the positively charged backbone is a nonpeptidyl polymer, which may be a hetero- or homo-polymer such as a polyalkyleneimine, for example a polyethyleneimine or polypropyleneimine, having a molecular weight of from about 10,000 to about 2,500,000, preferably from about 100,000 to about 1,800,000, and most preferably from about 500,000 to about 1,400,000. In another group of embodiments, the backbone has attached a plurality of side-chain moieties that include positively charged groups (e.g., ammonium groups, pyridinium groups, phosphonium groups, sulfonium groups, guanidinium groups, or amidinium groups). The sidechain moieties in this group of embodiments can be placed at spacings along the backbone that are consistent in separations or variable. Additionally, the length of the sidechains can be similar or dissimilar. For example, in one group of embodiments, the sidechains can be linear or branched hydrocarbon chains having from one to twenty carbon atoms and terminating at the distal end (away from the backbone) in one of the above-noted positively charged groups. In all aspects of the present invention, the association between the carrier and the therapeutic or cosmetic active agent is by non-covalent interaction, non-limiting examples of which include ionic interactions, hydrogen bonding, van der Waals forces, or combinations thereof.

In one group of embodiments, the positively charged backbone is a polypeptide having multiple positively charged sidechain groups (e.g., lysine, arginine, ornithine, homoarginine, and the like). Preferably, the polypeptide has a molecular weight of from about 10,000 to about 1,500,000, more preferably from about 25,000 to about 1,200,000, most preferably from about 100,000 to about 1,000,000. One of skill in the art will appreciate that when amino acids are used in this portion of the invention, the sidechains can have either the D- or L-form (*R* or *S* configuration) at the center of attachment. Alternatively, the backbone can be an analog of a polypeptide such as a peptoid. See, for example, Kessler, Angew. Chem. Int. Ed. Engl. 32:543 (1993); Zuckermann et al. Chemtracts-Macromol. Chem. 4:80 (1992); and Simon et al. Proc. Nat'l. Acad. Sci. USA 89:9367 (1992)). Briefly, a peptoid is a polyglycine in which the sidechain is attached to the backbone nitrogen atoms rather than the α-carbon atoms. As above, a portion of the sidechains will typically terminate in a positively charged group to provide a positively charged backbone component. Synthesis of peptoids is described in, for example, U.S. Pat. No. 5,877,278, which is hereby incorporated by reference in its entirety. As the term is used herein, positively charged backbones that have a peptoid backbone construction are considered "non-peptide" as they are not composed of amino acids having naturally occurring sidechains at the .alpha.-carbon locations.

A variety of other backbones can be used employing, for example, steric or electronic mimics of polypeptides wherein the amide linkages of the peptide are replaced with surrogates such as ester linkages, thioamides (--CSNH--), reversed thioamide (--NHCS--), aminomethylene (--NHCH₂--) or the reversed methyleneamino (--CH₂NH--) groups, keto-methylene (--COCH₂--) groups, phosphinate (--PO₂RCH₂--), phosphonamidate and phosphonamidate ester (--PO₂RNH--), reverse peptide(--NHCO--), trans-alkene (--CR=CH--), fluoroalkene (--CF=CH--), dimethylene (--CH₂CH₂--), thioether (--CH₂S--), hydroxyethylene (--CH(OH)CH₂--), methyleneoxy (--CH₂O--), tetrazole (CN₄), sulfonamido (--SO₂NH--), methylenesulfonamido (--CHRSO₂NH--), reversed sulfonamide (--NHSO₂--), and backbones with malonate and/or gem-diamino-alkyl subunits, for example, as reviewed by Fletcher et al. ((1998) Chem. Rev. 98:763) and detailed by references cited therein. Many of the foregoing substitutions result in approximately isosteric polymer backbones relative to backbones formed from α-amino acids.

In each of the backbones provided above, sidechain groups can be appended that carry a positively charged group. For example, the sulfonamide-linked backbones (--SO₂NH-- and --NHSO₂--) can have sidechain groups attached to the nitrogen atoms. Similarly, the hydroxyethylene (--CH(OH)CH₂--) linkage can bear a sidechain group attached to the hydroxy substituent. One of skill in the art can readily adapt the other linkage chemistries to provide positively charged sidechain groups using standard synthetic methods.

Optionally, the positively charged backbone is a polypeptide having efficiency groups. As used herein, an efficiency group is any agent that has the effect of promoting the translocation of the positively charged backbone through a tissue or cell membrane. Non-limiting examples of efficiency groups include -(gly)_{*n*1} -(arg)_{*n*2}, HIV-TAT or fragments thereof, or the protein transduction domain of Antennapedia, or a fragment thereof, in which the subscript n1 is an integer of from 0 to 20, more preferably 0 to 8, still more preferably 2 to 5, and the subscript n2 is independently an odd integer of from about 5 to about 25, more preferably about 7 to about 17, most preferably about 7 to about 13. Still further preferred are those embodiments in which the HIV-TAT fragment has the formula (gly)*ₚ*-RGRDDRRQRRR-(gly)*_{q}*, (gly)*ₚ*-YGRKKRRQRRR-(gly)*_{q}* or (gly)*ₚ*-RKKRRQRRR-(gly)*_{q}* wherein the subscripts p and q are each independently an integer of from 0 to 20 and the fragment is attached to the backbone via either the C-terminus or the N-terminus of the fragment. Preferred HIV-TAT fragments are those in which the subscripts p and q are each independently integers of from 0 to 8, more preferably 2 to 5. In some embodiments, the carrier has the amino acid sequence selected from the group consisting of RKKRRQRRR-G-(K)₁₅-G-RKKRRQRRR, RKKRRQRRR-G-(K)₂₀-G-RKKRRQRRR, RKKRRQRRR-G-(K)₂₅-G-RKKRRQRRR, RKKRRQRRR-G-(K)₃₀-G-RKKRRQRRR, RGRDDRRQRRR-G-(K)₁₅-G- RGRDDRRQRRR, RGRDDRRQRRR-G-(K)₂₀-G- RGRDDRRQRRR, RGRDDRRQRRR-G-(K)₂₅-G-RGRDDRRQRRR, RGRDDRRQRRR-G-(K)₃₀-G- RGRDDRRQRRR, YGRKKRRQRRR-G-(K)₁₅-G- YGRKKRRQRRR, YGRKKRRQRRR-G-(K)₂₀-G-YGRKKRRQRRR, YGRKKRRQRRR-G-(K)₂₅-G- YGRKKRRQRRR, and YGRKKRRQRRR-G-(K)₃₀-G- YGRKKRRQRRR.

In another preferred embodiment the positively charged efficiency group is the Antennapedia (Antp) protein transduction domain (PTD), or a fragment thereof that retains activity. (See, e.g., Console et al., J. Biol. Chem. 278:35109 (2003), the contents of which are incorporated by reference in their entirety.) Preferably the positively charged carrier includes side-chain positively charged efficiency groups in an amount of at least about 0.05%, as a percentage of the total carrier weight, preferably from about 0.05 to about 45 weight %, and most preferably from about 0.1 to about 30 weight %. For positively charged efficiency groups having the formula -(gly)_{*n*1}-(arg)_{*n*2}, the most preferred amount is from about 0.1 to about 25%.

In certain embodiments, the backbone portion is a polylysine and positively charged efficiency groups are attached to the lysine sidechain amino groups. In some embodiments, the polylysine may have a molecular weight that ranges from about 10,000 to about 1,500,000, preferably from about 25,000 to about 1,200,000, and most preferably from about 100,000 to about 1,000,000. In certain embodiments, the polylysine may have a molecular weight that ranges from about 500 to about 5000, about 1000 to about 4000, about 1500 to about 3500, or about 2000 to about 3000. The polylysine may be any of the commercially available (Sigma Chemical Company, St. Louis, Mo., USA) polylysines such as, for example, polylysine having MW>70,000, polylysine having MW of 70,000 to 150,000, polylysine having MW 150,000 to 300,000 and polylysine having MW>300,000. The selection of an appropriate polylysine will depend on the remaining components of the composition and will be sufficient to provide an overall net positive charge to the composition and, in some embodiments, provide a length that is preferably from one to four times the combined length of the negatively charged components. Preferred positively charged efficiency groups or efficiency groups include, for example, -gly-gly-gly-arg-arg-arg-arg-arg-arg-arg (-Gly₃Arg₇) or HIV-TAT. In another preferred embodiment the positively charged backbone is a long chain polyalkyleneimine such as a polyethyleneimine, for example, one having a molecular weight of about 1,000,000.

In certain embodiments, the carrier is a polylysine with positively charged branching groups attached to the lysine side-chain amino groups. The polylysine used in this particularly embodiment can be any of the commercially available (Sigma Chemical Company, St. Louis, Mo., USA, e.g.) polylysines such as, for example, polylysine having MW>70,000, polylysine having MW of 70,000 to 150,000, polylysine having MW 150,000 to 300,000 and polylysine having MW>300,000. However, preferably the polylysine has MW of at least about 10,000. Preferred positively charged branching groups or efficiency groups include, for example, -gly-gly-gly-arg-arg-arg-arg-arg-arg-arg (-Gly₃Arg₇), HIV-TAT or fragments of it, and Antennapedia PTD or fragments thereof.

In certain embodiments of this invention, the carrier is a relatively short polylysine or polyethyleneimine (PEI) backbone (which may be linear or branched) and which has positively charged branching groups. Such carriers are useful for minimizing uncontrolled aggregation of the backbones and botulinum toxin in a therapeutic composition, which causes the transport efficiency to decrease dramatically. When the carrier is a relatively short linear polylysine or PEI backbone, the backbone will have a molecular weight of less than 75,000, more preferably less than 30,000, and most preferably, less than 25,000. When the carrier is a relatively short branched polylysine or PEI backbone, however, the backbone will have a molecular weight less than 60,000, more preferably less than 55,000, and most preferably less than 50,000.

### Formulations

The topical compositions used in the methods for treating wrinkles described herein are typically in a form that permits the topical compositions to be applied onto and along the wrinkle distribution, such as, for example a liquid, cream, ointment, gel or lotion. In certain embodiments, the topical composition preferably is formulated to include a viscosity modifier that is capable of increasing the viscosity of the composition, so as to make the topical application easier and more accurate. For example, the viscosity modifying agent may be a gelling agent. Moreover, the viscosity modifying agent may be chosen to prevent the reconstituted composition from drying out, which can cause a decrease in the activity of chemodenervating agents, such as botulinum toxin. Particularly preferred viscosity modifying agents are those that are uncharged and do not interfere with the activity of the chemodenervating agent or the efficiency of the penetration of the chemodenervating agent upon administration of the topical composition. The viscosity modifying agents may contain cellulose-based gelling agents, a non-limiting example of which is a hydroxylalkylcellulose, such as hydroxypropylcellulose (HPC) or hydroxypropyl methylcellulose. In certain preferred embodiments, the topical compositions comprise 2-4% HPC. Alternatively, the viscosity modifying agent may be a polyalcohol, a non-limiting example of which is polyethylene glycol (PEG).

In some embodiments, the topical compositions are formulated to include a nonionic surfactant. Generally, this invention contemplates the use of any non-ionic surfactant that has the ability to stabilize the therapeutic agent or cosmetic agent (e.g., botulinum toxin) and that is suitable for pharmaceutical use. In certain embodiments, the non-ionic surfactant is a polysorbate, non-limiting examples of which include polysorbate 20, polysorbate 40, polysorbate 60, and polysorbate 80. In other embodiments, the non-ionic surfactant is a sorbitan ester, non-limiting examples of which include Span 20, Span 60, Span 65, and Span 80. The invention also contemplates using Triton X-100 or NP-40 as the non-ionic surfactants. In addition, the combinations of different non-ionic surfactants are contemplated. In certain preferred embodiments, the non-ionic surfactant is selected from the group consisting of polysorbates, poloxamers, and sorbitans, with polysorbates and sorbitans being particularly preferred. In preferred embodiments, the concentration of the non-ionic surfactant is in the range of 0.005% to 0.5%, or in the range of 0.01% to 0.2%, or in the range of 0.02% to 0.1% or in the range of 0.05 to 0.08%. This invention also contemplates formulations where the concentration of the non-ionic surfactant is 0.01%, 0.02%, 0.03%, 0.04%, 0.05%, 0.06%, 0.07%, 0.08%, 0.09%, 0.10%, 0.11%, 0.12%, 0.13%, 0.14%, or 0.15%.

While the topical compositions used in the methods described herein preferably are in the form of a liquid, cream, ointment, gel or lotion at the time of application, it is to be understood that the topical compositions may be in a different form during transportation or storage. For example, in some embodiments, the topical compositions are prepared in a solid form for ease of handling, transport, or storage. The solid form may be prepared by any method known in the art. Non-limiting examples of such methods include powder forms prepared by lyophilization, vacuum-drying, drum-drying or spray drying, with lyophilization and vacuum-drying being particularly preferred. When the chemodenervating agent is botulinum toxin, one particularly suitable powder form is the form described in U.S. Pre-Grant Publication No. 20100330123.

When the chemodenervating active agent is a proteinaceous material, it is often desirable to stabilize the chemodenervating active agent before, during, or after lyophilization by including a non-reducing sugar in the topical composition. Generally speaking, the non-reducing sugar may be any sugar with a glass transition temperature above 60° C. In certain particularly preferred embodiments, the non-reducing sugar is a disaccharide, non-limiting examples of which include trehalose and sucrose. In other embodiments, the non-reducing sugar is a trisaccharide, a non-limiting example of which is raffinose. Generally, the concentration of the non-reducing sugar in the topical formulations of the invention are in the range of 10% to 40%, preferably 10% to 25%, more preferably 15% to 20%. In some preferred embodiments, the concentration of the non-reducing sugar is 10%, 11%, 12%, 13%, 14%, 15%, 16%, 17%, 18%, 19% or 20%.

In certain embodiments, the topical compositions of the invention comprise a bulking agent that makes it easier to handle lyophilized forms of the topical composition. Preferably, the bulking agents crystallize under lyophilization conditions and do not mix well with the other excipients when in the solid state. Non-limiting examples of such bulking agents include sorbitol, mannitol, glycine, arginine, and histidine. The concentration of the bulking agent may be in the range of 1% to 10%, 2% to 6%, 3% to 5% or 4% to 4.5%. When a bulking agent is used, the concentration of the non-reducing sugar may be reduced from the 10% to 40% range to a range of 0.5% to 3.0%. Furthermore, in preferred embodiments, the ratio of the non-reducing sugar to the bulking agent is in the range of 0.07 to 2.0, preferably in the range of 0.4 to 0.6. Thus, by way of example only, the formulation may comprise mannitol as the bulking agent and trehalose as the non-reducing sugar, with mannitol present in a concentration range of 1.5% to 7.5% and trehalose present in a concentration range of 0.5% to 3.0%.

The diluent for reconstituting the topical compositions of the invention include any pharmaceutically or cosmetically acceptable diluent that is capable of reconstituting the topical composition. In certain embodiments, the diluent is simply water, saline, or a pharmaceutically acceptable buffer. Non-limiting examples of such buffers include those involving salts of citric acid, acetic acid, succinnic acid, tartaric acid, maleic acid, and histidine. Non-limiting examples of suitable buffer concentrations include buffer concentrations in the range of 0.400% to 0.600%; 0.450% to 0.575%, or 0.500% to 0.565%. The invention also contemplates diluents comprising a mixture of buffer salts, non-limiting examples of which include citrate/acetate, citrate/histidine, citrate/tartrate, maleate/histidine, or succinate/histidine. In certain preferred embodiments, the buffer is phosphate buffer.

In some embodiments, the diluent comprises a poloxamer, non-limiting examples of which include 101, poloxamer 105, poloxamer 108, poloxamer 122, poloxamer 123, poloxamer 124, poloxamer 181, poloxamer 182, poloxamer 183, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 331, poloxamer 333, poloxamer 334, poloxamer 335, poloxamer 338, poloxamer 401, poloxamer 402, poloxamer 403, and poloxamer 407. In certain preferred embodiments, the poloxamer is present in a concentration that ranges from 10-30%, or from 13-25%, or from 14-21%, or from 15-17%, or 16-16.5%.

As the skilled artisan will appreciate, the amount of chemodenervating agent present in a given topical composition will depend on several factors, including, for example, the specific chemodenervating agent chosen, the specific transdermal carrier chosen, and the desired effect. Generally speaking, the topical compositions will contain an effective amount of the chemodenervating agent. The term "effective amount" refers to an amount that is sufficient to achieve the desired result, but that is also implicitly safe (*i*.*e*., such that undesirable side effects, if present, are at an acceptable level). For example, when the chemodenervating agent is botulinum toxin type A, an effective amount may be in the range of 10 U to 150 U or 1,000 U to 2,500 U. In certain embodiments, the therapeutic amount ranges from 400 U to 800 U, or from 1,000 U to 50,000 U, preferably from 2,000-35,000 U, more preferably from 3,000 U-30,000 U, and even more preferably from 4,000 U to 25,000 U. In certain embodiments, the therapeutic amount ranges from 4,000 U to 8,000 U, 9,000 U to 19,000 U, or 20,000 U to 40,000 U. Of course, the specific amount of botulinum toxin type A in a given topical formulation will depend on the chosen transdermal carrier, and can be readily determined by a person of skill in the art.

### Treatment Regimens

The methods described herein can be incorporated into various treatment regimens. For example, the methods for treating wrinkles described herein may be performed on a given patient every one week, two weeks, three weeks, month, two months, three months, four months, five months, six months, seven months, eight months, nine months, ten months, eleven months or twelve months. The length of time between applications will vary depending on many factors, including the specific chemodenervating agent chosen, the initial dosage, and the rate at which wrinkles reappear in the areas that have been treated. In the case of botulinum toxin type A, for example, the method for treating wrinkles may be repeated every three or four months as needed.

### ASPECTS OF THE INVENTION

1. A method of reducing the appearance of wrinkles, the method comprising
   identifying a wrinkle distribution on a subject's skin, wherein the wrinkle distribution comprises one or more wrinkles, and
   applying a topical composition comprising at least one chemodenervating agent directly onto and along the wrinkle distribution to reduce the appearance of one or more wrinkles in the wrinkle distribution.
2. The method according to aspect 1, wherein the wrinkle distribution is present on the face, head, neck, hands, feet, shoulders, chest or back of the subject.
3. The method according to aspect 2, wherein the wrinkle distribution is present on the face of the subject.
4. The method according to aspect 3, wherein the wrinkle distribution comprises a lateral canthal line, a glabellar line, a forehead line, a platysma line, a nasolabial line or a perioral lip line.
5. The method according to aspect 4, wherein the wrinkle distribution comprises a lateral canthal line.
6. The method according to aspect 1, wherein the at least one chemodenervating agent is selected from the group consisting of botulinum toxin, saxitoxin, tetanus toxin, tetrodotoxin and combinations thereof.
7. The method according to aspect 6, wherein the chemodenervating agent is botulinum toxin.
8. The method according to aspect 7, wherein the botulinum toxin is selected from the group consisting of botulinum toxin A, B, C₁, D, E, F, and G.
9. The method according to aspect 8, wherein the botulinum toxin is botulinum toxin type A.
10. The method according to aspect 8, wherein the botulinum toxin is a 150 kD botulinum toxin type A neurotoxin molecule.
11. The method according to aspect 6, wherein the botulinum toxin is recombinant botulinum toxin.
12. The method according to aspect 11, wherein the botulinum toxin is a modified botulinum toxin.
13. The method according to aspect 1, wherein the topical composition comprises a positively charged polymeric carrier in an effective amount for transdermal administration of the chemodenervating agent.
14. The method according to aspect 11, wherein the positively charged polymeric carrier comprises a positively charged polypeptide or a positively charged non-peptidyl polymer, and
   and optionally at least one an efficiency group attached to the positively charged polypeptide or positively charged non-peptidyl polymer.
15. The method according to aspect 14, wherein the positively charged polypeptide is selected from the group consisting of polylysine, polyarginine, and polyornithine.
16. The method according to 14, wherein the positively charged polypeptide or positively charged non-peptidyl polymer has a molecular weight between 500 and 500,000 D.
17. The method according to aspect 16, wherein the positively charged polypeptide or positively charged non-peptidyl polymer has a molecular weight between 500 and 50,000 D.
18. The method according to aspect 17, wherein the positively charged polypeptide or positively charged non-peptidyl polymer has a molecular weight between 500 and 5,000 D.
19. The method according to aspect 14, wherein the efficiency group is selected from the group consisting of -(gly)ₙ₁-(arg)ₙ₂, (gly)ₚ-RGRDDRRQRRR-(gly)_{q}, (gly)ₚ-YGRKKRRQRRR-(gly)q, (gly)ₚ-RKKRRQRRR-(gly)_{q} and the Antennapedia protein transduction domain,
   wherein the subscripts p and q are each independently an integer of from 0 to 20,
   wherein the subscript n1 is independently an integer of from 1 to 8 and the subscript n2 is independently an odd number of from 7 to 17.
20. The method according to aspect 19, wherein the efficiency group is -(gly)_{*n*1}-(arg)_{*n*2}.
21. The method according to aspect 19, wherein the efficiency group is (gly)ₚRGRDDRRQRRR-(gly)_{q}.
22. The method according to aspect 19, wherein the efficiency group is (gly)ₚ-YGRKKRRQRRR-(gly)_{q}.
23. The method according to aspect 19, wherein the efficiency group is (gly)ₚ-RKKRRQRRR-(gly)_{q}.
24. The method according to aspect 19, wherein the efficiency group is the Antennapedia protein transduction domain.
25. The method according to aspect 1, further comprising the step of applying an occlusive dressing over the wrinkle distribution after application of the topical composition comprising at least one chemodenervating agent.
26. The method according to aspect 25, wherein the occlusive dressing comprises a polymeric sheet.
27. The method according to aspect 25, wherein the polymeric sheet comprises cellulosic fibers.
28. The method according to aspect 25, wherein the polymeric sheet comprises a polymer selected from the group consisting of a polyalkylene chloride, polylactic acid, polyisobutene, polyethylene-vinylacetate, Teflon, polyurethane, polyethylene, polypropylene, and polystyrene.
29. The method according to aspect 28, wherein the polymeric sheet comprises a polyethylene.
30. The method according to aspect 28, wherein the polymeric sheet comprises a polypropylene.
31. The method according to aspect 28, wherein the polymeric sheet comprises a polystyrene.
32. The method according to aspect 26 wherein the polymeric sheet does not contain adhesive.
33. The method according to aspect 26 wherein the polymeric sheet does contain adhesive.
34. The method according to aspect 33, wherein the adhesive is present only on a portion of the polymeric sheet.
35. The method according to aspect 25, wherein the occlusive dressing remains on the skin for a period of time sufficient to allow an effective amount of the chemodenervating agent to penetrate the skin.
36. The method according to aspect 35, wherein the period of time ranges from 5 minutes to 4 hours.
37. The method according to aspect 36, wherein the period of time ranges from 10 minutes to 2 hours.
38. The method according to aspect 37, wherein the period of time ranges from 15 minutes to an hour.
39. The method according to aspect 38, wherein the period of time is approximately 30 minutes.

## Claims

1. A cosmetic or non-therapeutic method of reducing the appearance of wrinkles, the method comprising
identifying a wrinkle distribution on a subject's skin, wherein the wrinkle distribution comprises one or more wrinkles, and
applying a topical composition comprising at least one chemodenervating agent and a positively charged carrier molecule directly onto and along the wrinkle distribution, such as along the contours of one or more wrinkles in the wrinkle distribution to reduce the appearance of one or more wrinkles in the wrinkle distribution.

2. The method according to claim 1, wherein the wrinkle distribution is present on the face, head, neck, hands, feet, shoulders, chest or back of the subject; preferably
wherein the wrinkle distribution comprises a lateral canthal line, a glabellar line, a forehead line, a platysma line, a nasolabial line or a perioral lip line; optimally
a lateral canthal line;
wherein the chemodenervating agent is not applied based on the anatomy of the
underlying muscles.

3. The method according to claim 1, wherein
(a) the at least one chemodenervating agent is selected from the group consisting of botulinum toxin, saxitoxin, tetanus toxin, tetrodotoxin and combinations thereof; or
(b) the botulinum toxin is recombinant botulinum toxin; or a modified botulinum toxin.

4. The method according to claim 3(a), wherein the chemodenervating agent is (preferably recombinant or modified) botulinum toxin; preferably
wherein the botulinum toxin is selected from the group consisting of botulinum toxin A, B, C₁, D, E, F, and G; more preferably
wherein the botulinum toxin is botulinum toxin type A; optimally
wherein the botulinum toxin is a 150 kD botulinum toxin type A neurotoxin molecule.

5. The method according to claim 1, wherein the topical composition comprises a positively charged polymeric carrier in an effective amount for transdermal administration of the chemodenervating agent.

6. The method according to claim 5, wherein the positively charged polymeric carrier comprises a positively charged polypeptide or a positively charged non-peptidyl polymer
and optionally at least one an efficiency group attached to the positively charged polypeptide or positively charged non-peptidyl polymer; preferably
wherein
(a) the positively charged polypeptide is selected from the group consisting of polylysine, polyarginine, and polyornithine; or
(b) the positively charged polypeptide or positively charged non-peptidyl polymer has a molecular weight between 500 and 500,000 D; or
(c) the efficiency group is selected from the group consisting of -(gly)ₙₗ-(arg)ₙ₂, (gly)ₚ-RGRDDRRQRRR-(gly)_{q}, (gly)ₚ-YGRKKRRQRRR-(gly)_{q}, (gly)ₚ-RKKRRQRRR-(gly)q and the Antennapedia protein transduction domain,
wherein the subscripts p and q are each independently an integer of from 0 to 20,
wherein the subscript n1 is independently an integer of from 1 to 8 and the subscript n2 is independently an odd number of from 7 to 17.

7. The method according to claim 6(b), wherein the positively charged polypeptide or positively charged non-peptidyl polymer has a molecular weight between 500 and 50,000 D; preferably
between 500 and 5,000 D.

8. The method according to claim 6(c), wherein the efficiency group is -(gly)_{*n*1}-(arg)_{*n*2}; or
(gly)ₚRGRDDRRQRRR-(gly)_{q}; or
(gly)ₚ-YGRKKRRQRRR-(gly)_{q}; or
(gly)ₚ-RKKRRQRRR-(gly)_{q}; or
the Antennapedia protein transduction domain.

9. The method according to claim 1, further comprising the step of applying an occlusive dressing over the wrinkle distribution after application of the topical composition comprising at least one chemodenervating agent.

10. The method according to claim 9, wherein the occlusive dressing comprises a polymeric sheet.

11. The method according to claim 10, wherein
(a) the polymeric sheet comprises cellulosic fibers; or
(b) the polymeric sheet comprises a polymer selected from the group consisting of a polyalkylene chloride, polylactic acid, polyisobutene, polyethylene-vinylacetate, Teflon, polyurethane, polyethylene, polypropylene, and polystyrene.

12. The method according to claim 11(b), wherein the polymeric sheet comprises a polyethylene; or
a polypropylene; or
a polystyrene.

13. The method according to claim 10, wherein the polymeric sheet
(a) does not contain adhesive; or
(b) contains an adhesive; preferably
wherein the adhesive is present only on a portion of the polymeric sheet.

14. The method according to claim 9, wherein the occlusive dressing remains on the skin for a period of time sufficient to allow an effective amount of the chemodenervating agent to penetrate the skin; preferably
wherein the period of time ranges from 5 minutes to 4 hours; more preferably
from 10 minutes to 2 hours; even more preferably
from 15 minutes to an hour; optimally
is approximately 30 minutes.

15. The use of a topical composition comprising at least one chemodenervating agent and a positively charged carrier molecule in a method of treatment comprising reducing the appearance of wrinkles, the method comprising
identifying a wrinkle distribution on a subject's skin, wherein the wrinkle distribution comprises one or more wrinkles, and
applying the topical composition directly onto and along the wrinkle distribution, such as along the contours of one or more wrinkles in the wrinkle distribution, to reduce the appearance of one or more wrinkles in the wrinkle distribution;
optionally wherein the chemodenervating agent is botulinum toxin and/or a ISOKD botulinum toxin type A neurotoxic molecule.
